# EUROPEAN PATENT APPLICATION

(11) **EP 2 147 683 A1**
(43) Date of publication of application: **27.01.2010**
(21) Application number: 08161192.3
(22) Date of filing: 25.07.2008
(51) Int. Cl.: A61K 47/48, A61P 35/00, A61P 25/00, A61P 29/00, A61P 37/00, A61P 3/00, A61P 31/00, A61P 9/00

(54) **Construct and method for the internalization of cargo molecules into a cell**

(71) Applicant: DKFZ Deutsches Krebsforschungszentrum, 69120 Heidelberg (DE)
(72) Inventor: The designation of the inventor has not yet been filed
(74) Representative: Dick, Alexander

(57) **Abstract**

The present invention relates to the delivery of molecules into cells. Especially, it relates to a carrier complex comprising (i) a molecule comprising a receptor peptide and a peptide which is capable of penetrating a host cell; and (ii) a second molecule comprising a ligand peptide and a cargo molecule of interest.
In a preferred embodiment, the carrier complex comprises (i) a fusion protein between the TAT-peptide of HIV I and the streptactin, a streptavidin mutant; and (ii) a conjugate or a fusion protein between the strep-tag II, a streptavidin, binding peptide, and the cargo molecule to be delivered.

SEQUENCE LISTING

<110>  Deutsches Krebsforschungszentrum, Stiftung des öffentlichen
        Rechts

<120>  Construct and method for the internalization of cargo molecules
       into a cell

<130>  DK65402EP

<160>  19

<170>  PatentIn version 3.4

<210>  1
<211>  9
<212>  PRT
<213>  artificial sequence

<220>
<223>  Strep-tag

<400>  1

Ala Trp Arg His Pro Gln Phe Gly Gly
1               5


<210>  2
<211>  9
<212>  PRT
<213>  artificial sequence

<220>
<223>  Nano-tag 9

<400>  2

Asp Val Glu Ala Trp Leu Gly Ala Arg
1               5


<210>  3
<211>  15
<212>  PRT
<213>  artificial sequence

<220>
<223>  Nano-tag 15

<400>  3

Asp Val Glu Ala Trp Leu Gly Ala Arg Val Pro Leu Val Glu Thr
1               5                   10                  15


<210>  4
<211>  38
<212>  PRT
<213>  artificial sequence

<220>
<223>  SBP-tag

<400>  4

Met Asp Glu Lys Thr Thr Gly Trp Arg Gly Gly His Val Val Glu Gly
1               5                   10                  15

Leu Ala Gly Glu Leu Glu Gln Leu Arg Ala Arg Leu Glu His His Pro
            20                  25                  30

Gln Gly Gln Arg Glu Pro
        35

<210>  5
<211>  8
<212>  PRT
<213>  artificial sequence

<220>
<223>  Strep-tag 2

<400>  5

Trp Ser His Pro Gln Phe Glu Lys
1               5

<210>  6
<211>  13
<212>  PRT
<213>  artificial sequence

<220>
<223>  TAT peptide

<400>  6

Tyr Gly Arg Lys Lys Arg Arg Gln Arg Arg Arg Pro Pro
1               5                   10

<210>  7
<211>  16
<212>  PRT
<213>  artificial sequence

<220>
<223>  Penetration peptide

<400>  7

Arg Gln Ile Lys Ile Trp Phe Gln Asn Arg Arg Met Lys Trp Lys Lys
1               5                   10                  15

<210>  8
<211>  7
<212>  PRT
<213>  artificial sequence

<220>
<223>  An7 peptide

<400>  8

Arg Arg Met Lys Trp Lys Lys
1               5

<210>  9
<211>  9
<212>  PRT
<213>  artificial sequence

<220>
<223>  polyarginine

<400>  9

Arg Arg Arg Arg Arg Arg Arg Arg Arg
1               5


<210>  10
<211>  21
<212>  PRT
<213>  artificial sequence

<220>
<223>  Buforin

<400>  10

Thr Arg Ser Ser Arg Ala Gly Leu Gln Phe Pro Val Gly Arg Val His
1               5                   10                  15


Arg Leu Leu Arg Lys
            20


<210>  11
<211>  26
<212>  PRT
<213>  artificial sequence

<220>
<223>  Transportan

<400>  11

Gly Trp Thr Leu Asn Ser Ala Gly Tyr Leu Leu Gly Lys Ile Asn Lys
1               5                   10                  15


Ala Leu Ala Ala Leu Ala Lys Lys Ile Leu
            20                  25


<210>  12
<211>  18
<212>  PRT
<213>  artificial sequence

<220>
<223>  MAP

<400>  12

Lys Leu Ala Leu Lys Leu Ala Leu Lys Ala Leu Lys Ala Ala Leu Lys
1               5                   10                  15


Leu Ala



<210>  13
<211>  16
<212>  PRT
<213>  artificial sequence

<220>
<223>  K-FGF

<400>  13

Ala Ala Val Ala Leu Leu Pro Ala Val Leu Leu Ala Leu Leu Ala Pro
1               5                   10                  15


<210>  14
<211>  5
<212>  PRT
<213>  artificial sequence

<220>
<223>  Ku 70 peptide

<400>  14

Val Pro Met Leu Lys
1               5


<210>  15
<211>  5
<212>  PRT
<213>  artificial sequence

<220>
<223>  Ku 70 peptide

<400>  15

Pro Met Leu Lys Glu
1               5


<210>  16
<211>  28
<212>  PRT
<213>  artificial sequence

<220>
<223>  Prion peptide

<400>  16

Met Ala Asn Leu Gly Tyr Trp Leu Leu Ala Leu Phe Val Thr Met Trp
1               5                   10                  15


Thr Asp Val Gly Leu Cys Lys Lys Arg Pro Lys Pro
            20                  25


<210>  17
<211>  18
<212>  PRT
<213>  artificial sequence

<220>
<223>  pVEC

<400>  17

Leu Leu Ile Ile Leu Arg Arg Arg Ile Arg Lys Gln Ala His Ala His
1               5                   10                  15

Ser Lys

<210>  18
<211>  21
<212>  PRT
<213>  artificial sequence

<220>
<223>  Pep 1

<400>  18

Lys Glu Thr Trp Trp Glu Thr Trp Trp Thr Glu Trp Ser Gln Pro Lys
1               5                   10                  15

Lys Lys Arg Lys Val
            20

<210>  19
<211>  18
<212>  PRT
<213>  artificial sequence

<220>
<223>  SynB1

<400>  19

Arg Gly Gly Arg Leu Ser Tyr Ser Arg Arg Arg Phe Ser Thr Ser Thr
1               5                   10                  15

Gly Arg

## Description

The present invention relates to the delivery of molecules into cells. Especially, it relates to a carrier complex comprising (i) a molecule comprising a receptor peptide and a peptide which is capable of penetrating a host cell; and (ii) a second molecule comprising a ligand peptide and a cargo molecule of interest.

The delivery of chemical compounds ("cargos") into cells is an important problem that has to be solved during the development of pharmaceutical compounds that act on intracellular targets. Often the pharmaceutical compound cannot penetrate the cell membrane, because it is too large or too hydrophilic. This is especially true for peptides and proteins that contain many polar or even charged side chains.

The chemical modification of cargos is one way to achieve membrane penetration. This is a laborious process. Alternatively a cargo, e.g. a peptide that is unable to penetrate the cell membrane can be replaced by a membrane penetrating small molecule. However, finding a membrane penetrating small molecule binding to the same target as the original compound requires an expensive and laborious screening process. Other methods for the internalization of cargos, such as transfection, can only be applied in a laboratory setting.

In another approach to this problem the cargos are fused to ligands that cause receptor mediated endocytosis of the fusion molecule. This method requires the attachment of a relatively large molecule to the cargo. The ligand-cargo fusion molecules, however, may have altered properties with respect to both, ligand affinity as well as cargo activities including the pharmaceutical properties. Furthermore, the biophysical characteristics of the cargo as a result of the linkage, like solubility, stability and folding for instance, may be changed.

One approach to find a generally applicable solution to the above described problem utilizes cell penetrating streptavidin derivatives as carriers. Because streptavidin binds biotin, these carriers function with every cargo that is biotinylated. However, the introduction of a biotinyl-residue into a molecule may drastically change its chemical and biophysical characteristics. Thus, biotinylation of a compound may impair its pharmaceutical properties. Moreover, chemical biotinylation may for some cargos be inefficient so that merely a small portion of the cargo will in fact become biotinylated.

The problem underlying the present invention could be seen as the provision of means and methods for the effective internalization of cargos, especially peptides and polypeptides, into host cells.

The present invention relates to a carrier complex comprising (i) a molecule comprising a receptor peptide and a peptide which is capable of penetrating a host cell; and (ii) a second molecule comprising a ligand peptide and a cargo molecule of interest.

A "receptor peptide" as referred to in the present application is a peptide capable of selectively binding the ligand peptide. Binding is selective if the ligand binds to the receptor with a significantly increased affinity compared to other molecules, preferably with at least 2 times, at least 5 times, at least 10 times, at least 20 times, at least 100 times, at least 500 times, at least 10⁴ times and most preferably at least 10⁶ times higher affinity than to other molecules. The affinity can be determined with well known techniques. The binding is, preferably, mediated by non-covalent interactions, such as hydrogen bonds, van-der-Waals forces or the electrostatic interaction of ions. In a preferred embodiment of the carrier complex of the present invention the receptor peptide is streptavidin. Streptavidin is a tetrameric polypeptide isolated from the bacterium *Streptomyces avidinii.* Native streptavidin can be proteolytically processed to a limited extent at its N- and C-terminus. To avoid a mixture of different degradation products, in general, a core fragment of streptavidin termed "core streptavidin", is recombinantly produced in *E.coli*. Recombinant core strepatvidin is described in DE000004135543. Core streptavidin is a tetrameric protein having a combined mass of approximately 53,000 Dalton. It comprises four identical subunits and binds biotin with very high affinity and specificity. One tetramer of streptavidin is capable of binding four molecules of biotin. Preferred nucleic acid sequences for core streptavidin are disclosed in DE000004135543. In another preferred embodiment of the carrier complex of the present invention said receptor is streptactin. Streptactin was derived from streptavidin. It displays an improved binding to Strep-tag II. From conventional streptavidin it differs in amino acid positions 44 to 47 (for preferred sequences see Skerra and Voß, DE000019641876).

A "ligand peptide" is characterized by its ability to selectively bind to the above described receptor molecules. Preferred ligand peptides are those which specifically bind to either streptavidin or streptactin. Thus, in a preferred embodiment of the carrier complex of the present invention said ligand peptide is Strep-tag, Nano-tag₉, Nano-tag₁₅, or SBP. Strep-tag is a peptide comprising 9 amino acids (AWRHPQFGG; SEQID No. 1) which binds with a high affinity to streptavidin (Skerra, 2003, Biospektrum 9: 189-192). If Strep-tag is used as ligand peptide, it is required that it is attached to the carboxy-terminus of the cargo polypeptide, because the binding of Strep-tag to streptavidin requires a free carboxy-terminus. Nano-tag₉ and Nano-tag₁₅ are peptides comprising 9 and 15 amino acids, respectively (DVEAWLGAR; SEQID No. 2; DVEAWLGARVPLVET; SEQID No. 3). Both peptides possess nanomolar-affmities for streptavidin (Lamla and Erdmann, 2004, Protein Expr. Purif. 33: 39-47). The SBP-tag is another streptavidin-binding peptide, composed of 38 amino acids, which binds with a nanomolar affinity to streptavidin (MDEKTTGWRGGHVVEGLAGELEQLRARLEHHPQGQREP; SEQID No. 4; Keefe et al., 2001, Protein Expr. Purif. 23: 440-446).

In another preferred embodiment of the carrier complex of the present invention said ligand peptide is Strep-tag II (WSHPQFEK; SEQID No. 5). This molecule was derived from Strep-tag and differs from it in a few positions. These amino acid exchanges lead to a lower affinity for streptavidin, but to a more than one order of magnitude stronger affinity for streptactin. In contrast to Strep-tag it can be used in any position in a peptide although it is advantageous to attach it at the termini of the cargo peptide to avoid interference with the normal peptide structure and its biochemical functions. Strep-tag II requires only a short two amino acid spacer between the cargo and the tag to ensure accessibility. Due to its chemically balanced amino acid composition, Strep-tag II does not interfere with folding or bioactivity of the cargo.

A "host cell" in the context of the present invention is a cell to which the cargo molecule of interest is directed. Preferably, it is a eukaryotic cell, more preferably, it is a eukaryotic cell that is part of a living organism. The said eukaryote cell can be a cell culture cell or a cell comprised by a living organism.

The term "peptide capable of penetrating a host cell" refers to a peptide that comprises an amino acid sequence which enables it to induce its own internalization into the cell. Preferably this sequence encodes a PTD (protein transduction domain) or the ligand of an endocytosis receptor. PTDs usually comprise between 5 and 35 amino acids. They often can be found e.g. in surface proteins of viruses, because viruses also have to be able to introduce their nucleic acids and regulatory proteins into the host cell. Other molecules that are bound to this molecule can often be internalized concomitantly. Preferably, the TAT-peptide of HIV I (YGRKKRRQRRRPP; SEQID No. 6; Frankel and Pabo, 1988, Cell 55: 1189-1193; Green and Loewenstein, 1988, Cell 55: 1179-1188), Penetratin and Ant7, derived from the homeodomain of the *Drosophila melanogaster* protein Antennapedia (RQIKIWFQNRRMKWKK; SEQID No. 7; Derossi et al., 1994, J Biol Chem. 269:10444-10450; RRMKWKK; SEQID No. 8, Honda et al., 2005, Front Biosci. 10:1290-301), or polyarginines (RRRRRRRRR; SEQID No. 9; Futaki et al., 2001, J Biol Chem. 276: 5836-5840) are used. Further preferred PTDs are Buforin II (TRSSRAGLQFPVGRVHRLLRK; SEQID No. 10), Transportan (GWTLNSAGYLLGKINKALAALAKKIL; SEQID No. 11), MAP (KLALKLALKALKAALKLA; SEQID No. 12), K-FGF (AAVALLPAVLLALLAP; SEQID No. 13), Ku70 (VPMLK; SEQID No. 14; PMLKE; SEQID No. 15), Prion (MANLGYWLLALFVTMWTDVGLCKKRPKP; SEQID No. 16), pVEC (LLIILRRRIRKQAHAHSK; SEQID No. 17), Pep-1 (KETWWETWWTEWSQPKKKRKV; SEQID No. 18), or SynB1 (RGGRLSYSRRRFSTSTGR; SEQID No. 19) (Joliot and Prochiantz, 2004, Nat Cell Biol. 6: 189-196). The term also refers to a ligand of endocytosis receptors which may serve as a peptide capable of penetrating a host cell. Endocytosis receptors are located in the cell membrane. They bind specifically to extracellular molecules. Upon binding an invagination of the cell membrane occurs around the receptor. Finally, the receptor, its ligand and all molecules in close proximity are internalized into small clathrin-coated vesicles. After internalization the vesicles shed their clathrin coat and fuse with other vesicles resulting in the early endosome. Preferred endocytosis receptors are the transferrin receptor, the LDL (low-density lipoprotein) receptor or the folate receptor.

The term "cargo molecule" refers to a molecule that is to be internalized into the host cell. This requires that it can be linked to the ligand peptide and that it does not interfere with the binding of the ligand peptide to the receptor peptide or with the internalization of the carrier complex into the host cell. Preferably, the ligand peptide and the cargo molecule are linked covalently. Preferably, the cargo molecule is a peptide, polypeptide, a nucleic acid, or a small molecule. Peptides or polypeptides as cargo molecules are discussed in detail below. Nucleic acids to be useful as a cargo molecule in accordance with the present invention include DNA molecules, RNA molecules or derivatives thereof. Preferably, DNA molecules may be oligonucleotides or polynucleotides such as cDNA or genomic DNA. RNA molecules, preferably, include microRNA, siRNA, mRNA, tRNA or ribozymes. A small molecule in accordance with the present invention is an organic or inorganic chemical compound. The small molecule may belong to any known chemical class of molecules. Preferably, small molecules are lipids, fatty acids, purines, pyrimidines, amino acids, biogenic amines, isoprenoids, or steroides.

Advantageously, the carrier complex of the present invention offers the opportunity to deliver diverse cargo molecules, preferably polypeptides, easily to target cells.

Known methods for the internalization of cargo molecules into cells comprise three steps: (i) the production of the cargo molecule, (ii) the linkage reaction of the cargo molecule with a ligand, typically biotin, and (iii) the combination of the complex of ligand and cargo molecule with a molecule that mediates the internalization of the formed complex. After the linkage of cargo molecule and ligand molecule a purification of the reaction product is required to remove substances that might interfere with administration of the complex, e.g. because they are toxic to the host. Furthermore, it cannot be excluded that the ligand molecule reacts with functional groups of the cargo molecule that are essential for its desired function in the cell.

If the cargo molecule is a polypeptide, the above described cumbersome and error prone process can be simplified. The molecule comprising a ligand peptide and a cargo molecule of the present invention can be formed in one single step by linking the polynucleotide sequence encoding the cargo polypeptide with the polynucleotide encoding the ligand peptide and expressing this recombinant polypeptide. There is no need for a separate chemical reaction that couples ligand molecule and cargo polypeptide. Thus, no purification step after coupling is necessary which saves labour. Furthermore, no undesired chemical modification of essential functional groups of the target molecule can occur during the linking reaction, because there is no such reaction. Finally, every single cargo molecule carries a ligand peptide. There is no loss of cargo molecules due to an incomplete linking reaction with the ligand.

The linkage of the ligand, typically biotin, to a cargo polypeptide often requires a spacer arm between the ligand and the polypeptide to enable receptor-binding by reducing steric hindrance. The known spacers, typically a 6 carbon spacer, often reduce the solubility of the cargo in aqueous media. To increase solubility, organic solvents or a medium comprising a substantial level of organic constituents is often required. However, the insolubility in aqueous solutions is problematic for *in vivo* applications where organic solvents cannot be used. The ligand peptides described above, typically Strep-tag II, require if at all only a short two amino acid spacer between the cargo and the tag to ensure accessibility. Due to their chemically balanced amino acid compositions they possess a high solubility in aqueous solutions.

Since the first molecule comprising a receptor peptide and a peptide which is capable of penetrating a host cell and the second molecule of the carrier complex comprising a ligand peptide and a cargo molecule can be produced separately, the biosafety is increased. The second molecule which has a biological function cannot penetrate into the host cell and the first molecule itself has, in general, no biological function. Unless the two molecules are combined, the cargo can not penetrate the host cell membrane.

As set forth above, in a preferred embodiment of the carrier complex of the present invention said cargo molecule is a polypeptide. The terms "polypeptide" and "protein" will be used interchangeably herein. If a polypeptide is used as a cargo, a ligand peptide can easily be linked to it. The nucleic acid sequence encoding the cargo polypeptide needs only to be linked to a nucleic acid sequence encoding the ligand peptide. This can be achieved with standard methods well known in the art (Sambrook, J. and Russell, D.W. "Molecular Cloning: A Laboratory Manual", 3d Edition, CSHL Press, 2001). Preferably, the ligand peptide is attached at one of the termini of the cargo polypeptide. In these positions it is least likely to interfere with the structure and the biochemical function of the cargo polypeptide. Such a nucleic acid construct can be cloned into a standard expression vector so that the recombinant fusion polypeptide can be produced in large amounts. Purification of the desired product poses no problems, because the ligand peptide can be used to purify it. A preferred method for the purification step is affinity chromatography using a column that is coated with the binding partner of the ligand peptide. After that step the purified fusion polypeptide is available for the binding to the carrier complex without further modification.

In another preferred embodiment of the carrier complex of the present invention said polypeptide is an intracellular signaling molecule, a transcription factor or a cytoskeleton protein. Preferably, a polypeptide to be used as a cargo according to the present invention shall have a therapeutic benefit including factors inducing cell death, growth arrest, senescence or immunostimulation and the like.

The term "intracellular signaling molecule" refers to a molecule that takes part in the intracellular transduction of signals. A signaling cascade starts with an intra- or extracellular stimulus. Intracellular stimuli, preferably, include DNA-damage and changed redox conditions. Extracellular stimuli, preferably, include the binding of ligands to receptors in the cell membrane. Often, the stimulus leads to the generation of a second messenger that starts the actual signaling cascade. The first activated intracellular signaling molecule usually activates other intracellular signaling molecules so that a whole signaling cascade is initiated. At the end of the cascade those molecules are activated that confer the physiological changes to the cell that can be observed as biological response. Intracellular signaling molecules are, preferably, polypeptides. Intracellular signaling molecules, preferably, are activated by the binding of second messengers, phosphorylation or proteolytic cleavage and use these mechanisms to activate their target cells. Second messengers are, preferably, calcium ions, nitric oxide, cyclic adenosine monophosphate (cAMP) or inositol triphosphate (IP₃).

The term "cytoskeleton protein" refers to proteins that are associated with the eukaryotic cytoskeleton. The cytoskeleton stabilizes the cell shape, enables cellular motion, is important for cell division and for intracellular transport processes. It is assembled from filaments. There are three main types of filaments in the cytoskeleton: microtubuli, actin filaments/microfilaments and intermediate filaments, each of which is composed of and associated with different proteins. Proteins associated with microtubuli comprise, preferably, α- and β-tubulin, dynein and kinesin. Proteins associated with actin filaments/microfilaments comprise, preferably, globular actin, α-actinin, capping protein and actin-related protein 2/3. Proteins associated with intermediate filaments comprise, preferably, keratins, desmin, GFAP (glial fibrillary acid protein), vimentin, α-intemexin, neurofilaments and lamins.

The term "transcription factor" is known to the person skilled in the art. It relates to a protein that is capable to increase or decrease the transcription of a gene by binding to the expression control sequence of the gene or to the RNA-polymerase complex.

In another preferred embodiment of the carrier complex of the present invention said host cell is a mammalian host cell. The host cell is preferably part of a cell or tissue culture, more preferably it is part of an intact organism. The mammalian host cell is, preferably, a primate or a rodent host cell, most preferably a human host cell.

In another preferred embodiment of the carrier complex of the present invention said host cell is a diseased host cell.

A "diseased host cell" in the context of the present application is a host cell whose physiological functions are impaired. Such an impairment can have different consequences. Preferably the cell loses one or more required functions, it gains one or more novel and unwanted functions, it excessively performs one or more of its normal functions or it displays a combination of any of the aforementioned conditions. Loss of function, preferably, includes hypoproliferation or the failure of a cell to produce a required molecule, such as a signaling molecule. Gain of function preferably includes hyperproliferation, excessive production of signaling molecules or excessive activity of cells of the immune system. Unwanted functions are all functions that are normally not exercised by the cell in question and whose exercise compromises the functioning or the well being of the organism. Diseases which are associated with impairment of physiological cellular functions are, preferably, neurological diseases, inflammatory diseases, allergic diseases, autoimmune diseases, deficiencies of the immune system, cancer, metabolic diseases, infectious diseases or cardiovascular diseases.

Neurological diseases include, preferably, Parkinson's disease, Alzheimer's disease or Huntington's disease. Metabolic diseases include, preferably, type 2 Diabetes.

In another preferred embodiment of the carrier complex of the present invention it is used as a medicament.

Preferably, the term "medicament" refers to the compound of the present invention, i.e. the above described carrier complex, and one or more pharmaceutically acceptable carrier. The compound of the present invention can be formulated as pharmaceutically acceptable salt. Acceptable salts comprise acetates, methylesters, sulfates, chlorides and the like. It is to be understood that the compound of the present invention comprises two molecules: (i) the fusion peptide comprising of a receptor peptide and a peptide which is capable of penetrating the host and (ii) comprises a ligand peptide and the cargo molecule. The former part of the compound is not responsible for the specific pharmaceutical effect of the medicament. It only secures the delivery of the second part of the carrier complex to the target cell or cells. Preferably, the cargo molecule that is part of the second part of the carrier complex causes the desired pharmaceutical effect of the medicament. Preferably, the pharmaceutical effect of the cargo is the reversal of the above described malfunctioning of a diseased cell. Also preferably, the cargo leads to the death of the target cell or cells.

The use of the compound of the present invention as medicament includes, preferably, the administration of the compound and the one or more pharmaceutically acceptable carriers to a patient. The compound and the carrier(s) are, preferably, administered topically or systemically. Suitable routes of administration conventionally used for drug administration are oral, intravenous, dermal or parenteral administration as well as inhalation. However, depending on the nature and mode of action said compound and the pharmaceutically acceptable carrier(s) may be administered by other routes as well.

In another preferred embodiment of the compound of the present invention it is used for the manufacture of a medicament for treating or preventing a disease or disorder associated with impaired intracellular functions.

The term "manufacture of a medicament" refers to all the steps necessary to produce a medicament. The compound of the present invention is, preferably, administered in conventional dosage forms prepared by combining the said compound with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is governed by the amount of active ingredient with which it is to be combined, the route of administration and other well-known variables. The carrier must be acceptable in the sense of being compatible with the other ingredients of the formulation and being not deleterious to the recipient thereof. The pharmaceutical carrier employed shall be, preferably, a solid, a gel or a liquid. Preferred solid carriers are lactose, terra alba, sucrose, talc, gelatin, agar, pectin, acacia, magnesium stearate, stearic acid and the like. Preferred liquid carriers are phosphate buffered saline solution, syrup, oil such as peanut oil and olive oil, water, emulsions, various types of wetting agents, sterile solutions and the like. Similarly, the carrier may include time delay material well known to the art, such as glyceryl mono-stearate or glyceryl di-stearate alone or with a wax. Further suitable carriers are well known in the art, see, e.g., Remington's Pharmaceutical Sciences, Mack Publishing Company, Easton, Pennsylvania.

The term "treating" refers to ameliorating the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

The term "preventing" refers to retaining health with respect to the diseases or disorders referred to herein for a certain period of time in a subject. It will be understood that the said period of time is dependent on the amount of the drug compound which has been administered and individual factors of the subject discussed elsewhere in this specification. It is to be understood that prevention may not be effective in all subjects treated with the compound according to the present invention. However, the term requires that a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed above.

The term "intracellular function" refers to a process that takes place inside a cell. Preferably, such a process enables the cell fulfill its role in the organism. Preferred intracellular functions are initiation of cell proliferation, initiation of cell differentiation, intracellular biosynthesis, preferably the production of signaling molecules, enzymes or antibodies, cell migration, cellular survival or any combination of the aforementioned functions.

Preferably, the cargo molecule is used as a therapeutic agent to improve impaired cellular functions or prevent their occurrence. In the case of diseases and disorders that are caused by a loss of physiological cellular functions, preferably, the carrier complex is used to administer a cargo molecule that restores the cellular function. If the loss of cellular function is caused by insufficient expression of a cellular protein, this is, preferably achieved, by administering the lacking protein or a nucleic acid encoding it to the cell or by administering a transcription factor that induces its expression. If the impaired cellular function is caused by a mutation that reduces the activity or expression of an essential protein, preferably, a functional version of said protein or a nucleic acid encoding it is administered to the cell with the aid of the cargo complex. In the case of diseases or disorders that are caused by unwanted cellular functions, a preferred treatment strategy aims to suppress these functions by inhibiting the expression or the activity of the responsible proteins. In this case the cargo molecule, preferably, binds specifically to the target protein and inhibits its function. Also preferably, the cargo molecule in such a case will be a transcription factor that reduces the expression of the protein or proteins that mediate the undesired cellular function.

In another preferred embodiment of the carrier complex of the present invention it is used for the manufacture of a medicament for treating or preventing cancer. The term "cancer", preferably, refers to solid cancers, preferably, carcinomas of the lung, breast, pharynx, oropharynx, laryngeal, penile, vulvar, pancreas, stomach, colon, testicles, skin, liver, kidney or to sarcomas. Also preferably, it refers to hematological cancers.

In another preferred embodiment of the carrier complex of the present invention it is used for the manufacture of a medicament for treating or preventing other diseases than cancer, preferably neurological diseases, inflammatory diseases, allergic diseases, autoimmune diseases, deficiencies of the immune system, metabolic diseases, infectious diseases, cardiovascular diseases.

Furthermore, the present invention also relates to a method for internalizing a polypeptide of interest into a host cell comprising
a) contacting said host cell with any of the carrier complexes described above, and
b) allowing internalization of the complex into the said host cell, whereby the polypeptide of interest is internalized.

The term "internalization into a host cell" refers to the uptake of the carrier complex of the present invention into the target cell. Preferably, the method is carried out in vitro, i.e. using isolated primary host cells or cell culture host cells. However, also preferably, the host cells can be comprised by an organism.

Furthermore, the present invention relates to a method for treating or preventing a disease or disorder associated with impaired intracellular functions comprising administering to a subject a therapeutically effective amount of any one of the carrier complexes of the present invention.

The term "therapeutically effective amount" refers to an amount of the compound to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of a population) and LD50 (the dose lethal to 50% of the population). The dose ratio between therapeutic and toxic effects is the therapeutic index, and it can be expressed as the ratio, LD50/ED50.

Furthermore, the present invention relates to a method for treating or preventing cancer comprising administering to a subject a therapeutically effective amount of any one of the carrier complexes of the present invention.

Finally, the present invention relates to a kit comprising a first molecule and a second molecule as described above or any of the carrier complexes of the present invention.

The term "kit" as used herein refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention is to be used for practicing the methods referred to herein above. It is, preferably, envisaged that all components are provided in a ready-to-use manner for practicing the methods referred to above. Further, the kit preferably contains instructions for carrying out the said methods. The instructions can be provided by a user's manual in paper form or electronic form.

All the documents cited in this application are hereby incorporated by reference.

The following examples illustrate the invention and are not intended to limit its scope in any way.

The Figures show:

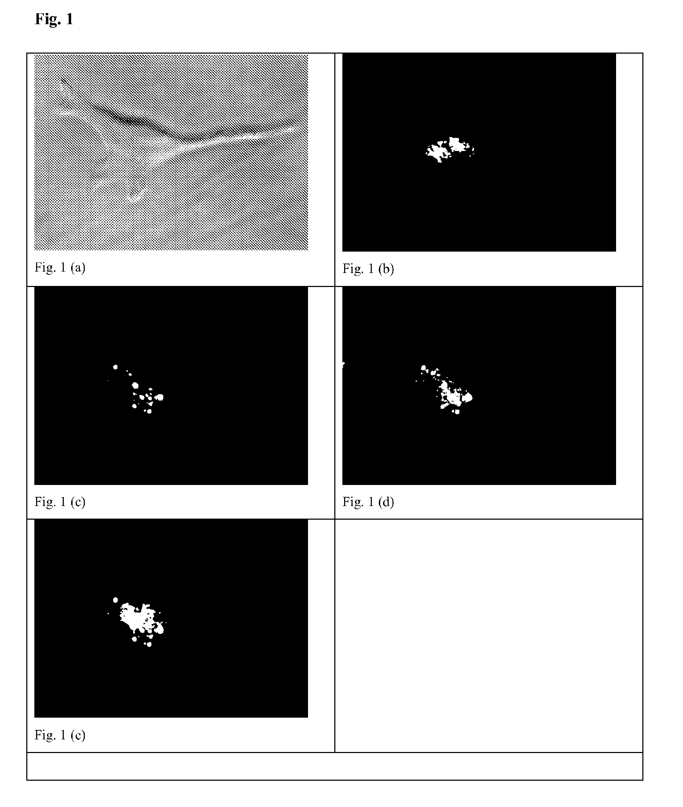

**Fig. 1** **Colocalization of internalized FITC-Strep-tag II by TAT-streptactin with an endocytic marker in fixed cells**
   (a) Microscopy image of a HeLa cell after treatment and fixation. (b) DNA-staining with DAPI. (c) FITC fluorescent signal of the FITC-Strep-tag II cargo. (d) Endosomes stained with TRITC-dextran. (e) Overlay of figures (b) to (d) shows colocalization of internalized FITC-Strep-tag II cargos by TAT-streptactin with TRITC-dextran.

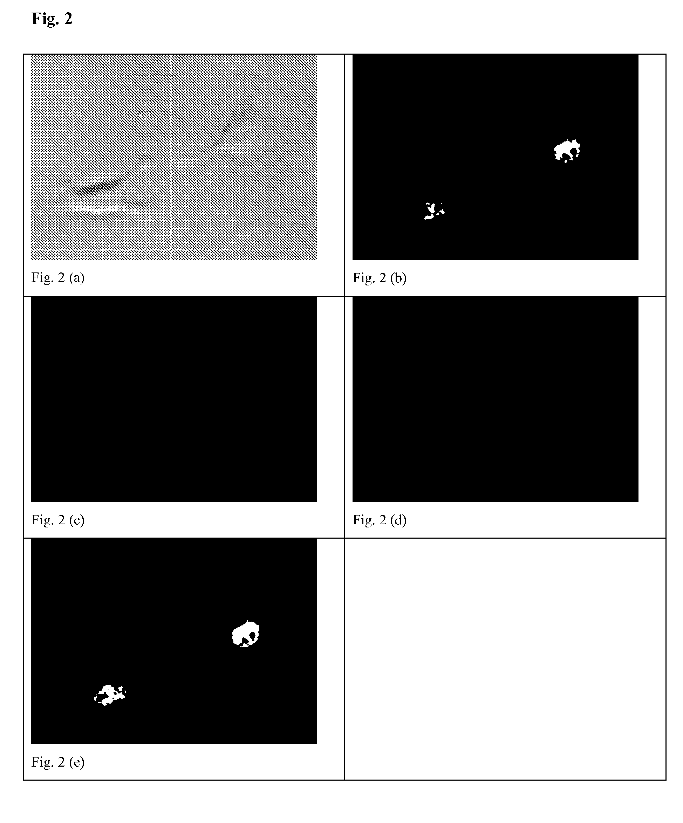

**Fig. 2** **No internalization of FITC-Strep-tag II by streptactin.**
   (a) Microscopy image of HeLa cells after treatment and fixation. (b) DNA-staining with DAPI. (c) FITC-fluorescent signal of the FITC-labeled Strep-tag II peptide. (d) TRITC-fluorescent signal of TRITC-labeled dextran. (e) Overlay of the figures (b) to (d) indicates no internalization of streptactin carrying FITC-Strep-tag II.

### Examples

### Example: Colocalization of internalized FITC-Strep-tag II by TAT-streptactin with an endocytic marker in fixed cells.

10µM TAT-streptactin was complexed with 10µM FITC-Strep-tag II peptide for 15 min at room-temperature. The complex was directly added to the medium of cultured HeLa cells. Endosomes resulting from the macropinocytic entry pathway were monitored with the fluid-phase endosomal marker TRITC-labeled dextran (250µg/mL medium). After 120 min, cells were washed twice, trypsinised, plated on glass cover-slips and after 4 hours fixed with paraformaldehyde. FITC- and TRITC-signals were monitored by fluorescent microscopy. In a control experiment the above described procedure was repeated with streptactin instead of TAT-streptactin.

As a result of the cell-permeable properties of the TAT protein transduction domain, TAT-streptactin caused the successful concomitant penetration of bound FITC-Strep-tag II model cargos across cellular membranes of cultured HeLa cells. Internalized FITC-Strep-tag II appeared in a punctuated fluorescent pattern around the cell nucleus which was identified as endosomal or endosomal-like compartments, due to the colocalization of FITC-Strep-tag II with TRITC-dextran as a fluid-phase endosomal marker. As negative control, HeLa cells treated with wildtype streptactin complexed with FITC-Strep-tag II showed only low or no fluorescent signals, in line with an insignificantly unspecific uptake. These results clearly demonstrate the potential of TAT-streptactin as a universal delivery system for Strep-tag II fused cargos.

## Claims

1. A carrier complex comprising:
a) a first molecule comprising a receptor peptide and a peptide which is capable of penetrating a host cell; and
b) a second molecule comprising a ligand peptide and a cargo molecule of interest.

2. The carrier complex of claim 1, wherein said receptor peptide is streptavidin.

3. The carrier complex of claim 1 or 2, when said ligand peptide is Strep-tag, Nano-tag₉, Nano-tag₁₅ or SBP.

4. The carrier complex of claim 1, wherein said receptor peptide is streptactin.

5. The carrier complex of claim 1 or 4, wherein said ligand peptide is Strep-tag II.

6. The carrier complex of any one of claims 1 to 5, wherein said cargo molecule of interest is a polypeptide.

7. The carrier complex of claim 6, wherein the said polypeptide is an intracellular signalling molecule, a transcription factor, or a cytoskeleton protein.

8. The carrier complex of any one of claims 1 to 7, wherein said peptide capable of penetrating a host cell is a protein transduction domain or a ligand of an endocytosis receptor.

9. The carrier complex of any one of claims 1 to 8, wherein said host cell is a mammalian cell.

10. The carrier complex of any one of claims 1 to 9, wherein said host cell is a diseased host cell.

11. A carrier complex of any one of claims 1 to 10 for use as a medicament.

12. Use of a carrier complex of any one of claims 1 to 10 for the manufacture of a medicament for treating or preventing a disease or disorder associated with impaired intracellular functions.

13. Use of a carrier complex of any of claims 1 to 10 for the manufacture of a medicament for treating or preventing cancer, neurological diseases, inflammatory diseases, allergic diseases, autoimmune diseases, deficiencies of the immune system, metabolic diseases, infectious diseases, cardiovascular diseases and the like.

14. A method for internalizing a polypeptide of interest into a host cell comprising
a) contacting said host cell with a carrier complex of any one of claims 1 to 10; and
b) allowing internalization of the complex into said host cell, whereby the polypeptide of interest is internalized.

15. A kit comprising a first molecule and a second molecule as defined in any one of claims 1 to 10 or the carrier complex of any one of claims 1 to 10.
